(19) Europäisches Patentamt
European Patent Office
Office européen des brevets

(11) **EP 2 571 376 B1**

(12) **FASCICULE DE BREVET EUROPEEN**

(45) Date de publication et mention
de la délivrance du brevet:
**23.04.2014 Bulletin 2014/17**

(21) Numéro de dépôt: **11727213.8**

(22) Date de dépôt: **16.05.2011**

(51) Int Cl.:
*A23L 1/00* $^{(2006.01)}$      *A23L 1/305* $^{(2006.01)}$

(86) Numéro de dépôt international:
**PCT/FR2011/051093**

(87) Numéro de publication internationale:
**WO 2011/144856 (24.11.2011 Gazette 2011/47)**

(54) **PROCEDE DE PREPARATION D'HYDROLYSATS ALCALINS DE PROTEINES VEGETALES**

VERFAHREN FÜR DIE ZUBEREITUNG VON ALKALISCHEN HYDROLYSATEN AUS PFLANZENPROTEINEN

METHOD FOR PREPARING ALKALINE HYDROLYSATES OF PLANT PROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.05.2010 FR 1053944**

(43) Date de publication de la demande:
**27.03.2013 Bulletin 2013/13**

(73) Titulaire: **Roquette Freres**
**62136 Lestrem (FR)**

(72) Inventeurs:
• **DHALLEINE, Claire**
**F-60200 Compiègne (FR)**
• **DELEPIERRE, Sophie**
**F-59890 Quesnoy sur Deule (FR)**

(74) Mandataire: **Cabinet Plasseraud**
**52, rue de la Victoire**
**75440 Paris Cedex 09 (FR)**

(56) Documents cités:
**GB-A- 705 489      US-A- 2 999 753**
**US-A- 5 274 079**

**Description**

La présente invention a pour objet un procédé de préparation d'hydrolysats alcalins de protéines végétales.

La présente invention est également relative aux hydrolysats alcalins de protéines végétales, caractérisés par leur solubilité à pH 7,5, leur longueur moyenne de chaîne peptidique, leur pouvoir émulsifiant à pH 7 et leur richesse.

Les protéines et les hydrolysats de protéines végétales ou animales sont fréquemment utilisés en tant qu'agents moussants dans les produits alimentaires, notamment en confiserie :

- les protéines en tant que telles, choisies comme agents moussants stables dans le temps,
- les hydrolysats de protéines, pour leur capacité moussante plus élevée que les protéines.

De nombreux documents décrivent les propriétés moussantes des hydrolysats de protéines. Les plus récents traitent de l'hydrolyse des protéines par voie enzymatique tandis que les plus anciens décrivent l'hydrolyse alcaline des protéines.

Par exemple, les hydrolysats alcalins des protéines du lait ont été décrits pour leur utilisation dans les mousses d'extincteur ou comme substitut des protéines d'oeuf dans les produits aérés alimentaires.

Dans le brevet US 2.522.050, il est décrit un procédé de fabrication d'agents moussants par l'hydrolyse alcaline d'une protéine de soja ou de lait dans une solution aqueuse contenant de l'hydroxyde de calcium ou de l'hydroxyde de magnésium à un pH d'au moins 10 et à une température dite bien inférieure à 100°C (35 - 40°C) pendant une longue période (au moins deux jours) afin d'obtenir un produit ayant des propriétés moussantes satisfaisantes.

Il est ainsi à noter que ce document recommande :

- de choisir des températures de réaction inférieures ou égales à 40°C,
- de préférer des hydroxydes de calcium ou de magnésium, et surtout des hydroxydes de calcium afin d'obtenir les hydrolysats de protéines présentant les meilleures propriétés moussantes,
- de privilégier de longues durées de réaction.

C'est ainsi que dans le brevet GB 670.413, il est décrit un procédé pour la préparation d'agents moussants par hydrolyse de protéines à température ambiante pendant une période d'au moins 24 heures, hydrolyse réalisée à l'aide d'un hydroxyde de calcium.

Il est également mentionné dans ce brevet que s'il est possible d'hydrolyser les protéines à une température plus élevée, de l'ordre de 100°C et plus, cela se fera au détriment des propriétés moussantes recherchées.

Cependant, si l'hydrolyse à l'hydroxyde de calcium est souvent conseillée, elle génère des hydrolysats de très mauvais goût, ce qui constitue un sérieux handicap. En général ils sont en effet crayeux et amers, et en outre ont un goût soufré et caoutchouteux.

L'augmentation de la température pendant l'hydrolyse à l'hydroxyde de calcium peut réduire le temps de réaction, mais augmente la formation de ces arômes indésirables.

Pour tenir compte de tous ces pré-requis, le brevet EP 1.327.390 propose alors une méthode pour aérer un produit alimentaire contenant des hydrates de carbone en utilisant un hydrolysat de protéine végétale comme agent moussant, hydrolysat obtenu en soumettant cette protéine végétale à une hydrolyse dans une solution aqueuse avec un pH d'au moins 10.

Cet hydrolysat alcalin présente alors une longueur moyenne de chaîne peptidique de 5 à 20 acides aminés et une quantité d'acides aminés libres inférieure à 15 % en poids de la matière totale dérivée des protéines.

Cependant, pour parvenir à ce résultat, le procédé original d'hydrolyse alcaline décrit dans le brevet EP 1.327.390 nécessite de combiner des hydroxydes alcalins et des hydroxydes alcalinoterreux, i.e. la combinaison d'au moins un hydroxyde alcalin tel que NaOH ou KOH avec au moins un hydroxyde alcalinoterreux, par exemple $Ca(OH)_2$ ou $Mg(OH)_2$.

Une hydrolyse alcaline efficace, selon les termes de ce brevet EP 1.327.390, ne peut donc être obtenue que par une conduite d'hydrolyse tout à fait particulière.

Dans la demande de brevet internationale WO 95/25437, il est décrit une méthode pour produire des hydrolysats de protéines végétales avec une coloration améliorée par l'extraction des protéines contenues dans des farines végétales à un pH au-delà du pH isoélectrique de la protéine, éventuellement en présence d'adsorbants, et l'hydrolyse de la protéine ainsi obtenue en présence d'adsorbants avec des alcalis, des acides et / ou des enzymes de manière dite « connue en soi ».

Les hydrolysats de protéines ainsi obtenus peuvent alors être utilisés notamment comme agents tensio-actifs.

Le procédé recommandé pour l'hydrolyse alcaline consiste en fait à traiter une fois encore la suspension aqueuse alcaline des isolats de protéines avec des oxydes ou hydroxydes de calcium.

Il faut alors filtrer la solution obtenue pour éliminer les résidus.

Pour obtenir les peptides en tant que tels, il faut encore traiter les peptides sous forme de sels de calcium avec de la soude ou de la potasse, puis éliminer le calcium résiduel, par exemple sous forme de sulfate de calcium.

La séparation des sels peu solubles doit finalement être effectuée en présence d'adjuvants de filtration sur filtre et filtres-presses.

Les hydrolysats ainsi obtenus, après concentration, ont un poids moléculaire moyen variant de 100 à 30 000 daltons, de préférence 100 à 10 000 daltons et surtout de 2000 à 5000 daltons et une teneur en matière sèche de 5 à 50 % en poids.

Dans le brevet EP 1.909.592, il est décrit un procédé pour la production d'hydrolysats de protéines enrichis en manganèse, destinées en zootechnie comme sources contrôlées d'apports en manganèse, permettant ainsi d'éviter le surdosage dans le régime alimentaire pour l'animal, et de réduire tous les phénomènes d'interférence avec d'autres composants de la ration alimentaire.

Pour obtenir ces hydrolysats de protéines riches en manganèse, il était déjà décrit dans l'état de la technique le traitement, par exemple à la chaux dans certaines conditions de pression et de température, des tissus conjonctifs dérivant de la peau traitée dans une tannerie.

Le brevet EP 1.909.592 propose plutôt d'obtenir les hydrolysats de protéines enrichis en manganèse en employant, comme matière de départ, une matière organique végétale classique, et en la soumettant notamment à un traitement avec de la chaux.

L'enrichissement en manganèse de ces protéines est alors effectué par le traitement des sels de calcium des hydrolysats de protéines avec des sulfates de manganèse ou des sels autres de manganèse à températures élevées, solubilisées préalablement dans des solutions d'acide sulfurique.

Il faut encore précipiter les sels de calcium résiduels par du bicarbonate d'ammonium, bicarbonate de sodium ou directement avec du dioxyde de carbone et/ou des agents précipitants autres, comme, par exemple, l'acide oxalique et l'acide phosphorique.

Dans la demande de brevet internationale WO 2008/001183, il est décrit des aliments présents sous forme de bâtonnets, de crackers ou de produits d'extrusion, riches en hydrolysats de protéines d'origine animale ou végétale, additionnés ou mélangés avec un excipient ou un diluant alimentaire.

Ces hydrolysats de protéines végétales ou animales présentent une teneur en protéines égale ou supérieure à 60 % en poids.

Les hydrolysats sont produits à partir de protéines végétales, animales ou de fermentation.

Il s'agit ici de peptides ou de polypeptides majoritairement solubles dans l'eau dans une gamme de pH allant de 3 à 11.

Les hydrolysats selon la demande WO 2008/001183 sont préférentiellement produits par action d'enzymes protéolytiques.

Le poids moléculaire des produits d'hydrolyse est compris entre 200 et 100 000 Daltons avec une préférence pour des poids moléculaires compris entre 200 et 20 000 Daltons.

Dans la demande de brevet WO 2007/079458, il est proposé de substituer des protéines aux matières grasses et aux carbohydrates. Mais sous leur forme native, les protéines ont des propriétés physiques et organoleptiques qui leur donnent un très mauvais goût.

Pour rendre les protéines plus attrayantes comme substitut de matières grasses, les protéines natives sont hydrolysées en peptides et polypeptides, principalement à l'aide d'enzymes de type protéases.

Ces fragments de protéines sont plus solubles dans l'eau. Ils peuvent être alors incorporés dans les boissons (obtention de boissons dites « concentrées en protéines »), ou additionnés aux nourritures solides pour leur conférer un goût moins crayeux.

Mais l'hydrolyse enzymatique des protéines natives a cependant un inconvénient important : les hydrolysats de protéines restent très amers et ne sont pas tous thermostables.

Pour réduire l'amertume, de nombreuses solutions ont été proposées, mais restent dans l'ensemble peu satisfaisantes.

Par exemple, il a été essayé de prolonger l'hydrolyse des protéines, de manière à obtenir de très courts peptides, des dipeptides, voire des acides aminés libres.

L'hydrolyse poussée des protéines permet de réduire l'amertume de façon significative, mais amène des goûts « savonneux ». Par ailleurs, l'arrière-goût amer et crayeux persiste même après l'hydrolyse.

Il est alors recommandé dans la demande WO 2007/079458 de revenir au procédé plus traditionnel de l'hydrolyse alcaline, mais de la coupler à une réaction d'hydrolyse enzymatique.

Cette double réaction d'hydrolyse conduit alors à l'obtention de courtes chaînes peptidiques.

Le procédé décrit dans cette demande consiste en effet à préparer une solution de protéines, à régler le pH de la solution à une valeur de 10,4 ou plus pour former une solution basique de protéines, et y ajouter une protéase.

Les hydrolysats de protéines ainsi obtenus ont alors un très faible poids moléculaire, en moyenne de 2000 à 10 000 Daltons.

Ces compositions peuvent être thermostables à une température d'au moins 87,8°C (190°F) pendant au moins 5 minutes, et présentent une amertume réduite.

Quant à la demande de brevet WO 2008/110515, il y est décrit une composition de protéines de céréales partiellement hydrolysées.

Ces hydrolysats de protéines présentent alors entre 20% et 80% en poids de protéines partiellement hydrolysées d'au plus 25 000 daltons et de l'ordre de 8% en poids de protéines partiellement hydrolysées d'au plus 1 400 Da.

Cette répartition en poids particulière, i.e. une fraction relativement haute en poids moléculaire, confère à ces hydrolysats partiels de protéines de céréales des propriétés comparables aux protéines du lait, avec moins d'inconvénients que ceux des protéines laitières.

De plus, une telle protéine partiellement hydrolysée de céréales présente une texture agréable dans la bouche et est facilement digestible.

Cependant, la matière de base est préférentiellement du gluten de blé vital, et le procédé demande un contrôle fin de l'hydrolyse par voie enzymatique de manière à atteindre un degré d'hydrolyse de 3 à 8.

Cette hydrolyse partielle est habituellement effectuée en présence d'une ou plusieurs enzymes exo- et endo-peptidases.

Enfin, le document GB 705 489 décrit l'hydrolyse de protéines de cacahuètes par de la soude, à 82°C pendant 30 minutes ; neutralisation à l'HCl et obtention d'hydrolysats « foisonnants », tandis que le document US 2 999 753 divulgue des hydrolysats alcalins de protéines végétales obtenus après traitement à 37 - 80°C (100-175°F) pendant 8 à 20 heures à un pH de 10,7-10,8.

Il résulte de tout ce qui précède qu'il demeure encore un besoin de disposer d'hydrolysats de protéines de pois, de pomme de terre et de maïs présentant une excellente solubilité, une répartition en poids moléculaires et un degré d'hydrolyse leur permettant d'exprimer un excellent pouvoir émulsifiant ainsi que de bonnes propriétés organoleptiques.

Par ailleurs, ces hydrolysats doivent pouvoir être produits par un procédé peu coûteux et simple à mettre en oeuvre, en d'autres termes économiquement et industriellement viable.

L'invention a donc pour but de remédier aux inconvénients des hydrolysats et procédés de l'état de la technique et la société Demanderesse a eu le mérite de trouver, après de nombreux travaux, que ce but pouvait être atteint en proposant un procédé particulier de préparation d'hydrolysats alcalins de protéines végétales.

Ce procédé de préparation d'hydrolysats alcalins de protéines végétales selon l'invention comprend les étapes suivantes :

1) préparer une suspension de protéines végétales choisies dans le groupe constitué des protéines du pois, des protéines de la pomme de terre et des protéines du maïs, à une matière sèche comprise entre 10 et 15%,

2) ajuster le pH, sous agitation, à une valeur comprise entre 9,5 et 10,5 en utilisant, comme seul agent alcalin, un ou plusieurs hydroxydes alcalins choisis dans le groupe constitué de l'hydroxyde de sodium et de l'hydroxyde de potassium,

3) chauffer la suspension ainsi obtenue à une température comprise entre 70 et 80°C, pendant 4 à 6 heures,

4) neutraliser ladite suspension chauffée à l'aide d'un acide minéral, de préférence de l'acide chlorhydrique,

5) sécher la suspension neutralisée de manière à obtenir l'hydrolysat alcalin.

La première étape du procédé permettant d'obtenir des hydrolysats alcalins conformes à l'invention consiste à préparer une suspension de protéines végétales à une matière sèche (ci-après « MS ») comprise entre 10 et 15%.

La deuxième étape du procédé permettant d'obtenir des hydrolysats alcalins conformes à l'invention consiste à ajuster le pH de la suspension de protéines végétales présentant de 10 à 15% de MS, sous agitation, à une valeur comprise entre 9,5 et 10,5, de préférence à une valeur de 10,0 avec de l'hydroxyde de sodium ou de l'hydroxyde de potassium.

L'ajustement du milieu réactionnel à un pH de l'ordre de 10 (ce qui peut se traduire par exemple par un apport en soude l'ordre de 1,2% en poids sec/poids sec de protéines de pois) permet d'obtenir les produits présentant le meilleur comportement en termes de solubilité et de capacité émulsifiante (ci-après « CE »).

La société Demanderesse a par ailleurs constaté que ces valeurs de solubilité et de CE n'évoluent plus de manière significative après 6 heures de réaction.

Quant à la basicité, elle est amenée uniquement par des hydroxydes de métaux alcalins de préférence des hydroxydes de sodium (NaOH) ou des hydroxydes de potassium (KOH).

On n'utilisera donc pas des hydroxydes de métaux alcalinoterreux tel que l'hydroxyde de calcium.

La troisième étape du procédé consiste à chauffer la suspension à une température comprise entre 70 et 80°C, pendant 4 à 6 heures.

Testée de 55 à 90°C, la température de réaction a été finalement choisie à une valeur comprise entre 70 et 80°C, de préférence de l'ordre de 75°C.

Quant à la durée de la réaction, elle est fixée entre 4 et 6 heures.

La société Demanderesse en procédant de la sorte va à l'encontre de préjugés de l'état de la technique, au sens où :

- le temps de réaction est très court, facilement industrialisable : il n'est donc plus nécessaire d'effectuer la réaction en 24 à 48 heures, voire plus,
- il n'est pas proposé d'utiliser des hydroxydes de calcium ou de magnésium. Au contraire, la société Demanderesse a trouvé que l'usage de la chaux impactait négativement la qualité des hydrolysats de protéines obtenus.

La quatrième étape du procédé permettant d'obtenir des hydrolysats alcalins conformes à l'invention consiste à neutraliser le pH à l'aide d'un acide minéral, de préférence de l'acide chlorhydrique.

Par exemple, de l'acide chlorhydrique à 1N est ajouté au milieu, sous agitation, afin d'ajuster le pH à 7.

La cinquième étape du procédé permettant d'obtenir des hydrolysats alcalins conformes à l'invention consiste à sécher l'hydrolysat alcalin ainsi obtenu.

Par exemple, le produit est séché sur un atomiseur à turbine de type NIRO fonctionnant en co-courant. Cet atomiseur ne possède pas de système de recyclage des fines ; il s'agit donc d'un séchage en simple effet. L'air entrant dans la tour d'atomisation est chauffé à 180°C. Le débit d'alimentation de la tour est réglé de telle manière à avoir l'air en sortie de tour à une température de l'ordre de 80 à 85°C. Ces conditions d'atomisation conduisent à une poudre ayant 6 à 7% d'humidité résiduelle.

La mise en oeuvre du procédé selon l'invention permet d'obtenir des hydrolysats alcalins de protéines végétales présentant de remarquables caractéristiques fonctionnelles.

Ces hydrolysats alcalins de protéines végétales sont ainsi caractérisés par :

- une valeur de solubilité dans l'eau à pH 7,5 comprise entre 60 et 100%, de préférence entre 80 et 98%,
- une capacité émulsifiante comprise entre 60 et 90%, de préférence 65 et 85%,
- une longueur moyenne de chaîne peptidique comprise entre 10 et 20 aminoacides,
- une richesse comprise entre 60 et 95%, préférentiellement comprise entre 80 et 85%.

Les hydrolysats alcalins conformes à l'invention sont caractérisés par leur solubilité, déterminée selon un test A.

Ce test A consiste à déterminer la teneur en matières solubles dans l'eau à pH 7,5 par une méthode de dispersion d'une prise d'essai de l'échantillon dans de l'eau distillée et analyse du surnageant obtenu après centrifugation.

Ainsi, il peut notamment être conduit de la manière suivante. Dans un bêcher de 400 ml, on introduit une prise d'essai d'exactement 2 g d'échantillon et un barreau aimanté (référencé par exemple sous le N°ECN 442-4510 par la société VWR). On tare le tout puis on ajoute 100 g d'eau distillée à 20°C $\pm$ 2°C.

On ajuste le pH à 7,5 avec HCl 1N ou NaOH 1N et on complète à 200 g exactement avec de l'eau distillée.

On agite pendant 30 minutes puis centrifuge 15 minutes à 3000g.

Après centrifugation, on prélève exactement 25 g de surnageant dans un cristallisoir préalablement taré. On place à l'étuve à 103°C jusqu'à masse constante.

La solubilité aqueuse est calculée grâce à l'équation suivante :

$$\text{Solubilité} = \frac{(m1-m2) \times 200 \times 100}{25 \times 2}$$

avec ml = masse en g du cristallisoir après séchage
m2 = masse en g du cristallisoir vide

Les hydrolysats alcalins conformes à l'invention présentent alors une solubilité comprise entre 60 et 100%, de préférence comprise entre 80 et 98%.

Les hydrolysats alcalins conformes à l'invention sont également caractérisés par leur capacité émulsifiante, déterminée selon un test B.

Ce test consiste à déterminer la Capacité Emulsifiante (ci-après « CE ») correspondant au pourcentage de « crème » d'émulsion formée stable après centrifugation en fonction d'une certaine concentration en protéines et en huile, à l'aide d'un homogénéisateur, tel que celui de marque POLYTRON et de type PT 45-80 (muni de préférence d'une broche Easy-clean référence B99582 / société Bioblock).

De manière plus précise, ce test consiste :

- Dans un pot de 2L de forme haute (i.e. présentant par exemple 23,5 cm de haut et 11,5 cm de diamètre), préparer une solution d'hydrolysats alcalins de protéines équivalant à 2,0 % de protéines (masse/volume en protéines N x 6,25) dans 250 ml d'eau déminéralisée,
- Introduire un barreau aimanté (référencé notamment sous le N°ECN 442-4510 par la société VWR),
- mélanger les hydrolysats alcalins de protéines pendant 10 minutes sur un agitateur magnétique, par exemple de marque IKA® RCT Classic, à la vitesse maximale de 1100 t/min,
- Préparer 250 ml d'huile de colza alimentaire,
- Retirer le barreau aimanté,
- Plonger la broche de l'homogénéisateur dans la solution, à mi-hauteur de la solution d'hydrolysats alcalins de protéines.
- Positionner la vitesse de rotation entre 15 200 et 15 450 tours/min,
- Lancer l'agitation et verser les 250 ml d'huile de colza en 1 minute,
- Transvaser l'émulsion dans un bécher,
- Peser 2 fois exactement 35 g de l'émulsion dans 2 tubes gradués à centrifuger de 50 ml,
- Centrifuger à 1500 g pendant 5 minutes, à 20°C,
- Mesurer le volume de là « crème » d'émulsion après centrifugation,
- Mesurer le volume total après centrifugation (culot + eau + crème d'émulsion),
- Vérifier la répétabilité entre les 2 tubes et entre 2 essais identiques.

La Capacité Emulsifiante sera déterminée par calcul, grâce à l'équation suivante :

$$CE = \frac{\text{Volume de crème d'émulsion après centrifugation}}{\text{Volume total après centrifugation}} \times 100$$

Les hydrolysats alcalins conformes à l'invention présentent alors une valeur de CE comprise entre 60 et 90 %, de préférence 65 et 85 %.

Les hydrolysats alcalins conformes à l'invention sont également caractérisés par leur longueur moyenne de chaîne peptidique, déterminée selon un test C.

Ce test C consiste à calculer la longueur moyenne de chaîne comme suit, où

- NT = azote total
- NAT = azote aminé total
- AAL = acides aminés libres
- F = teneur moyenne en azote des acides aminés de la protéine considérée
- LMCP = longueur moyenne des chaînes peptidiques
- AAP = nombre d'acides aminés peptidiques
- CP = nombre de chaînes peptidiques

NT est alors déterminé selon la méthode de Dumas A., 1826, Annales de chimie, 33, 342, comme cité par BUCKEE, 1994, dans Journal of the Institute of BREWING, 100, pp 57-64, méthode connue de l'homme de l'art et exprimé en mmol/g.

NAT est déterminé par formol titration dite de Sorensen, connue également de l'homme de l'art et exprimé en mmol/g.

AAL est déterminé par HPLC et exprimé en mmol/g.

Selon les protéines considérées, la valeur de F (exprimée en mol/mol) est la suivante :

- protéines de pois : 1,29
- protéines de pomme de terre : 1,25
- protéines de maïs: 1,24

La longueur moyenne de chaînes est égale au nombre d'acides aminés peptidiques divisé par le nombre de chaînes peptidiques, soit :

$$LMCP = \frac{AAP}{CP} \qquad avec : AAP = \left(\frac{NT}{F}\right) - AAL$$

$$et \quad CP = NAT - F \times AAL$$

Les hydrolysats alcalins conformes à l'invention présentent alors une longueur moyenne de chaîne peptidique comprise entre 10 et 20 aminoacides, ce qui traduit le caractère partiellement hydrolysé des protéines.

Enfin, les hydrolysats alcalins selon l'invention sont caractérisés par leur richesse (exprimée en Nx6,25), qui peut être déterminée selon une méthode bien connue de l'homme du métier.

Les hydrolysats alcalins de protéines conformes à l'invention sont également caractérisés par :

- leur qualité organoleptique,
- leur capacité moussante (ci-après : « CM »), et.
- leur degré d'hydrolyse.

La qualité organoleptique des hydrolysats alcalins conformes à l'invention a été déterminée notamment sur les hydrolysats alcalins de protéines de pois.

Les hydrolysats alcalins des protéines de pois conformes à l'invention présentent en effet une qualité organoleptique tout à fait satisfaisante, en comparaison avec les protéines de pois à partir desquelles ils sont préparés.

Comme il sera exemplifié ci-après, un profil sensoriel a été réalisé par la société Demanderesse de la façon suivante : les échantillons sont préparés dans des fioles en verre coloré à raison de 5 g de produit dans 150 g d'eau et maintenus à 50°C, ils sont ensuite présentés en aveugle aux panélistes.

Les panélistes doivent ensuite sentir et gouter le produit et cocher les cases correspondantes aux descripteurs.

Ce profil montre que la flaveur des hydrolysats alcalins de l'invention est différente des protéines de pois.

Selon le panel d'experts, que ce soit sur le critère olfactif ou sur le critère gustatif, les descripteurs « pois », mais aussi « acide », « amer », « âcre », « piquant », et « fermenté » des hydrolysats sont atténués par rapport à ceux des protéines de pois.

La capacité moussante est quant à elle déterminée selon le test D de la manière suivante.

Une mousse est une dispersion de bulles de gaz (azote, gaz carbonique, air) dans une phase continue (renfermant des protéines ou leurs hydrolysats) liquide ou solide produite par agitation mécanique.

Dans un bécher de 250 ml de forme haute (i.e. présentant par exemple 12 cm de haut et 6 cm de diamètre), on prépare une solution de 40 ml à 2% (masse/volume en protéines Nx6,25) des hydrolysats de protéines avec de l'eau déminéralisée.

On introduit un barreau aimanté (référencé notamment sous le N°ECN 442-4510 par la société VWR).

On hydrate les hydrolysats de protéines pendant 10 minutes sur un agitateur magnétique, tel que celui de marque IKA® RCT Classic, à la vitesse de 1100 tours/min.

On retire le barreau aimanté.

On mesure le volume total avant foisonnement.

On plonge la broche (par exemple référence G45M) d'un homogénéisateur, tel que celui de marque IKA® Werke de type ULTRA TURRAX® T50 basic, dans la solution d'hydrolysats de protéines à mi-hauteur de ladite solution.

On positionne la vitesse de rotation à environ 15.200 tours/min (c'est-à-dire sur la position « 5 » dans le cas de l'ULTRA TURRAX) et on laisse sous agitation 1 minute.

On transvase la totalité du volume dans une éprouvette graduée de 100 ml.

On mesure le volume total après foisonnement.

La capacité moussante est alors donnée par la formule suivante :

$$CM = \frac{\text{Volume total de mousse après foisonnement}}{\text{Volume total avant foisonnement}} \times 100$$

La perte de stabilité est exprimée par la perte de volume de mousse après 30 minutes, exprimée en pourcentage du volume de mousse initial.

Les hydrolysats alcalins conformes à l'invention présentent alors une valeur de CM comprise entre 150 et 250%.

Ces hydrolysats alcalins présentent par ailleurs un degré d'hydrolyse (DH) avantageusement compris entre 5 et 9. Celui-ci peut être déterminé par calcul, selon la formule suivante :

$$DH = [(NAT \%) \times 100]/ [Azote\ protéique]$$

où :

NAT est l'azote aminé total déterminé par formol titration dite de Sorensen, connue de l'homme de l'art, et exprimé en mmol/g,

l'azote protéique est exprimé en N x 6,25, et mesuré selon la méthode bien connue de l'homme du métier.

Les hydrolysats alcalins conformes à l'invention peuvent être avantageusement utilisés comme agents émulsifiants dans les domaines des industries alimentaires humaines ou animales, pharmaceutiques, cosmétiques et industries chimiques, en particulier dans le domaine alimentaire.

Ils peuvent également être utilisés dans les industries de la fermentation, des matériaux de construction, des matières plastiques, des matières textiles, du papier et du carton.

Enfin, la présente invention a pour objet des compositions, de préférence des compositions alimentaires, contenant les hydrolysats alcalins tels que décrits ci-dessus.

Ces compositions alimentaires sont de préférence des émulsions émulsifiées avec lesdits hydrolysats alcalins.

D'autres caractéristiques et avantages de l'invention apparaîtront à la lecture des exemples non limitatifs décrits ci-dessous.

EXEMPLES

**Exemple 1 : Préparation d'hydrolysats de protéines de pois**

Pour préparer les hydrolysats alcalins de protéines de pois conformes à l'invention, on procède de la manière suivante :

1) mettre en suspension 25 kg de protéines à 93% de MS soit 23,15 kg de MS dans 210 kg d'eau,

2) ajuster le pH à 10 avec de l'hydroxyde de sodium, et chauffer la suspension ainsi obtenue à une température de 75°C, pendant 4 heures,

3) neutraliser à pH = 7 avec de l'acide chlorhydrique 1 N,

4) atomiser l'hydrolysat alcalin ainsi obtenu.

Le produit est séché sur un atomiseur à turbine de type NIRO fonctionnant en co-courant. Cet atomiseur ne possède pas de système de recyclage des fines ; il s'agit donc d'un séchage en simple effet. L'air entrant dans la tour d'atomisation est chauffé à 180°C. Le débit d'alimentation de la tour est réglé de telle manière à avoir l'air en sortie de tour à une température de l'ordre de 80 à 85°C.

Ces conditions d'atomisation conduisent à produire une poudre ayant de l'ordre de 6% d'humidité résiduelle.

Les résultats obtenus figurent dans le tableau I suivant :

Tableau I

|  | Protéine native avant hydrolyse | Hydrolysat selon l'invention |
|---|---|---|
| Matière sèche (%) | 92,4 | 94,1 |
| Solubilité pH 7,5 (%) | 70,1 | 93,0 |
| CE (%) | 67,0 | 80,0 |
| Longueur moyenne de chaîne peptidique | 16 | 14 |
| Degré d'hydrolyse | 4,9 | 5,6 |
| Richesse (%) | 85,2 | 81,2 |
| CM (% d'augmentation de volume après foisonnement par rapport au volume initial) | 183 | 225 |

Les protéines de pois hydrolysées selon l'invention possèdent une longueur moyenne de chaîne peptidique de 14.

L'hydrolyse des protéines de pois selon l'invention permet d'augmenter :

- la solubilité de 70,1 à 93 %,
- la capacité émulsifiante de 67 à 80 %.

De plus, le pouvoir moussant est amélioré.

Les hydrolysats de protéines de pois selon l'invention présentent des propriétés de solubilité, de capacités émulsifiantes et moussantes supérieures aux propriétés des mêmes protéines avant hydrolyse.

## Exemple 2 : Hydrolyse de protéines de pois de haute richesse

Le mode opératoire décrit dans l'exemple 1 est appliqué à des protéines de pois ayant une teneur en protéines supérieure à 90 %.

Ces conditions opératoires produisent une poudre ayant les caractéristiques suivantes (tableau II):

Tableau II

| | Protéine native avant hydrolyse | Hydrolysat selon l'invention |
|---|---|---|
| Matière sèche (%) | 94,8 | 94,1 |
| Solubilité pH 7,5 (%) | 32 | 79,2 |
| CE pH 7 | 75 | 81 |
| Longueur moyenne de chaîne peptidique | 19 | 13 |
| Degré d'hydrolyse | 4 | 6 |
| Richesse (%) | 92,2 | 89,6 |

Les protéines de pois ayant une richesse supérieure à 90% lorsque hydrolysées selon l'invention possèdent une longueur moyenne de chaîne peptidique de 13.

L'hydrolyse des protéines de pois selon l'invention permet d'augmenter :

- la solubilité de 32 à 79,2%,
- la capacité émulsifiante de 75 à 81%.

## Exemple 3 : Préparation d'hydrolysats de protéines de pomme de terre

Le mode opératoire décrit dans l'exemple 1 est appliqué à des protéines de pomme de terre.

Ces conditions opératoires produisent une poudre ayant les caractéristiques suivantes (tableau III) :

Tableau III

| | Protéine native avant hydrolyse | Hydrolysat selon l'invention |
|---|---|---|
| Matière sèche (%) | 91,6 | 92,7 |
| Solubilité pH 7,5 (%) | 31,0 | 85,2 |
| CE pH 7 (%) | 60 | 67 |
| Longueur moyenne de chaîne peptidique | 13 | 13 |
| Degré d'hydrolyse | 6,2 | 8,0 |
| Richesse (%) | 70,7 | 65,3 |

De manière surprenante et inattendue, on constate donc que l'hydrolyse alcaline, sans impacter sur la longueur moyenne de chaîne peptique (valeur égale à 13 dans les deux cas), conduit cependant à un hydrolysat présentant une bien meilleure solubilité et une capacité émulsifiante améliorée.

L'hydrolyse des protéines de pomme de terre selon l'invention permet d'augmenter

- la solubilité de 31,0 à 85,2%,
- la capacité émulsifiante est de 60 à 67%.

**Exemple 4** : **Préparation d'hydrolysats de protéines de maïs**

Le mode opératoire décrit dans l'exemple 1 est appliqué à des protéines de maïs. Ces conditions opératoires produisent une poudre ayant les caractéristiques suivantes (tableau IV):

Tableau IV

|  | Protéine native avant hydrolyse | Hydrolysat selon l'invention |
|---|---|---|
| Matière sèche (%) | 95,1 | 91,4 |
| Solubilité pH 7,5 (%) | 5,1 | 97,4 |
| CE pH 7 (%) | 0 | 67 |
| Longueur moyenne de chaîne peptidique | 59 | 16 |
| Degré d'hydrolyse | 1,4 | 5,9 |
| Richesse (%) | 86,5 | 81 |
| CM (% d'augmentation de volume après foisonnement par rapport au volume initial) | 0 | 250 |

Les protéines de maïs hydrolysées selon l'invention possèdent une longueur moyenne de chaîne peptidique de 16.
L'hydrolyse des protéines de maïs selon l'invention permet d'augmenter :

- la solubilité de 5,1 à 97,4%,
- la capacité émulsifiante de 0 à 67%.

De plus, les protéines de maïs natives ne présentent aucun pouvoir moussant alors que les protéines de maïs hydrolysées selon l'invention possèdent de remarquables capacités moussantes.
Les hydrolysats des protéines de maïs selon l'invention permettent d'améliorer les propriétés de solubilité, de capacités émulsifiante et surtout moussante.

**Exemple 5** : **Tableau comparatif de différents hydrolysats de protéines végétales conformes à l'invention**

Tableau V

|  | Origine botanique des protéines hydrolysées | | |
|---|---|---|---|
|  | **Pois** | **Pomme de terre** | **Maïs** |
| Richesse (%) | 81,2 | 65,3 | 81 |
| Degré d'hydrolyse | 5,6 | 8,0 | 8,4 |
| Longueur moyenne de chaîne peptidique | 14 | 13 | 16 |
| % solubilité | 93,0 | 85,2 | 97,4 |
| CE (%) | 80,0 | 67,0 | 67,0 |
| CM : % d'augmentation de volume après foisonnement | 225 | 150 | 250 |

Quelle que soit l'origine botanique des protéines (pois, pomme de terre, maïs), l'hydrolyse selon l'invention permet d'obtenir des protéines ayant :

- une longueur moyenne de chaîne peptidique comprise entre 10 et 20 acides aminés,

- une solubilité de 85,2 à 97,4%,
- une capacité émulsifiante de 67 à 80%,
- une capacité moussante, définie par le pourcentage d'augmentation de volume après foisonnement, de 150 à 250%.

**Exemple 6 : Utilisation des hydrolysats selon l'invention pour l'encapsulation d'huiles**

De l'huile de poisson est encapsulée par atomisation d'une émulsion à 45% de MS et à pH = 8.

L'huile représente 15% de la matière sèche, le support d'encapsulation et l'émulsifiant varient selon les formules.

L'émulsion est réalisée selon le mode opératoire suivant :

- Dissoudre le support d'encapsulation et l'émulsifiant dans de l'eau déminéralisée chauffée à 80°C (= solution encapsulante)
- ajuster le pH à 8 à l'aide de NaOH 1N
- agiter pendant 20 minutes
- peser l'huile 5 minutes avant la fin de cette période pour éviter l'oxydation
- Réaliser l'émulsion à l'aide d'un homogénéisateur POLYTRON de type PT 45-80 (muni d'une broche Easy-clean référencée B99582 chez Bioblock), vitesse de 9000 rpm : pour cela, verser l'huile dans la solution encapsulante (préparée aux étapes 1 et 2) sous agitation en 2 minutes.
- Passer l'émulsion obtenue sur homogénéisateur haute pression à 160 Bar (30 Bar au 2ème étage et compléter jusqu'à 160 Bar pour le premier étage)
- Placer alors l'émulsion en agitation en maintenant la température proche de 50°C

L'émulsion ainsi préparée est atomisée sur un atomiseur simple effet (sans recyclage des fines particules). La température de l'air entrant est à 185°C ; le débit est réglé de façon à avoir T° sortie = 90°C.

Les poudres obtenues sont caractérisées par leur teneur en eau, l'activité de l'eau (aw), le taux d'encapsulation et par l'état d'oxydation de l'huile.

Le taux d'encapsulation est mesuré par différence entre la matière grasse totale et la matière grasse extractible (quantité d'huile fixée par le support) :

$$\text{Taux d'encapsulation (\%)} = 100 - \left[ \frac{\text{\% lipides extractibles}}{\text{\% lipides totaux}} \right] \times 100$$

Les lipides sont déterminés par extraction au SOXHLET à l'hexane :

- Sur le produit tel quel pour les lipides extractibles,

- Sur le produit après hydrolyse pour les lipides totaux.

La stabilité à l'oxydation est déterminée selon la norme NF ISO 6886.

Le temps d'induction correspond au temps nécessaire pour oxyder un corps gras dans des conditions données (température, débit d'air, masse de produit).

**Exemple 6.1** :

Atomisation d'émulsions à 45 % de MS et pH = 8 contenant :

- 15% d'huile de poisson,

- 1,2 ou 1,8 % d'émulsifiant : protéine de pois native / hydrolysat de protéines de pois de l'exemple 1,

- respectivement 83,5 % ou 83,2% de support : maltodextrine d'un DE 12 (GLUCIDEX® 12 commercialisé par la société ROQUETTE FRERES).

Les poudres atomisées ont une activité de l'eau de 0,1.

Leur teneur en eau est de 5% pour les essais avec 1,2% d'émulsifiant et de 4% pour les essais avec 1,8% d'émulsifiant.

Tableau VI : taux d'encapsulation (%) et temps d'induction (h) des émulsions atomisées avec la maltodextrine de DE 12.

Tableau VI

| Nature et teneur en émulsifiant | Taux d'encapsulation (%) | Temps d'induction (h) |
|---|---|---|
| 1,2% Protéines de pois natives | 78,3 | 6,0 |
| 1,2% Hydrolysats de protéines de pois conformes à l'invention | 87,9 | 8,5 |
| 1,8% Protéines de pois natives | 83,2 | 6,7 |
| 1,8% hydrolysats de protéines de pois conformes à l'invention | 90,0 | 11,0 |

Avec le support maltodextrine de DE 12, l'utilisation de l'hydrolysat de protéines de pois conforme à l'invention à hauteur de 1,2% permet d'encapsuler jusqu'à 87,9% d'huile contre 78,3% avec la protéine de pois native.

L'huile a alors un temps d'induction de 8,5 h contre 6 h.

Pour les deux concentrations d'émulsifiant, le taux d'encapsulation est supérieur lorsqu'on utilise l'hydrolysat de protéines de pois conforme à l'invention plutôt que la protéine de pois native.

De même, le temps d'induction est supérieur lorsqu'on utilise l'hydrolysat de protéines de pois conforme à l'invention plutôt que la protéine de pois native.

L'huile s'oxyde donc moins rapidement.

**Exemple 6.2** :

Atomisation d'émulsions à 45% de MS et pH = 8 contenant :

- 15% d'huile
- 1,2 ou 1,8% d'émulsifiant : protéine de pois native / hydrolysat de protéines de pois conforme à l'invention
- respectivement 83,8% ou 83,2% de support : dextrine de pois (TACKIDEX® C760 commercialisée par la société ROQUETTE FRERES)

Les poudres atomisées ont une activité de l'eau de 0,1.

Les teneurs en eau sont comparables pour une teneur en émulsifiant donnée.

Tableau VII : taux d'encapsulation (%) et temps d'induction (h) des émulsions atomisées avec la dextrine de pois.

Tableau VII

| Nature et teneur en émulsifiant | Teneur en eau | Taux d'encapsulation (%) | Temps d'induction (h) |
|---|---|---|---|
| 1,2% Protéines de pois natives | 4,6 | 62,0 | 6,1 |
| 1,2% Hydrolysats de protéines de pois conformes à l'invention | 4,2 | 82,9 | 7,7 |
| 1,8% Protéines de pois natives | 3,6 | 48,0 | 6,0 |
| 1,8% hydrolysats de Protéines de pois conformes à l'invention | 4,0 | 88,9 | 8,5 |

Avec les dextrines de pois comme support d'encapsulation et quelle que soit la teneur en émulsifiant, le taux d'encapsulation est supérieur lorsqu'on utilise l'hydrolysat de protéines de pois conforme à l'invention plutôt que la protéine de pois native.

De même, le temps d'induction est supérieur lorsqu'on utilise l'hydrolysat de protéines de pois conforme à l'invention plutôt que la protéine de pois native.

L'utilisation en tant qu'émulsifiant de l'hydrolysat de protéines de pois conforme à l'invention permet d'augmenter le taux d'encapsulation en comparaison avec la protéine de pois native.

De plus, l'utilisation de l'hydrolysat de protéines de pois conforme à l'invention permet d'augmenter le temps d'induction des huiles.

En d'autres termes, les huiles sont mieux protégées de l'oxydation lorsqu'on réalise l'émulsion avec l'hydrolysat de protéines de pois conforme à l'invention plutôt qu'avec la protéine de pois native.

**Exemple 7** : **Production de perméats de lactosérum ré-engraissés**

Les quantités de lactosérum disponibles dans le monde sont considérables puisqu'elles représentent au moins 85% du lait transformé en fromage.

Il existe de nombreuses utilisations possibles du sérum dans l'alimentation humaine et animale mais désormais la tendance est au fractionnement du lactosérum.

En particulier, les concentrés de protéines sériques trouvent un usage particulièrement adapté en alimentation infantile.

Les protéines sont séparées du lactosérum par ultrafiltration ce qui conduit à des volumes importants de perméats de lactosérum contenant principalement du lactose, des minéraux et des petites protéines solubles.

L'enjeu est de valoriser cette fraction soluble déprotéinée.

On se propose donc de ré-enrichir la fraction soluble issue de l'ultrafiltration du lactosérum :

- en matières grasses, à un niveau plus ou moins équivalent à celui du lait de vache, et
- en protéines, à des fins nutritionnelles et fonctionnelles, celles-ci permettant de stabiliser l'émulsion formée.

Enfin ce mélange est séché pour faciliter sa conservation. Les poudres sont principalement utilisées dans les aliments d'allaitement pour veaux.

On teste donc la capacité des hydrolysats de protéines de pois à remplacer avantageusement les protéines dans cette application.

Pour cela, on choisit :

- une protéine de pois native,
- un hydrolysat de protéines de pois conforme à l'invention (celui de l'exemple 1).

Les suspensions sont préparées à 60% de MS dont 40% de matières grasses et 16% de protéines par rapport à la formule hors gras.

Les formules sont préparées de la façon suivante : de l'eau est chauffée et maintenue à 50°C dans une cuve munie d'une double enveloppe.

Le perméat de lactosérum est ajouté ainsi que la source protéique.

Le pH du mélange est alors rectifié à 7 avec de l'hydroxyde de sodium.

La matière grasse liquide est ensuite ajoutée.

Le mélange est homogénéisé sur un homogénéisateur haute pression à deux étages, le premier étage est réglé à 20 Bar et le second étage à 80 Bar.

Le produit est alors séché sur une tour d'atomisation de type NIRO à buses et sans recyclage des fines.

Les conditions d'atomisation sont inchangées pour les deux formules : la température de l'air entrant est de 165°C, celle de l'air sortant de 94°C.

La pression d'atomisation est de l'ordre de 175 Bar. La température de l'air entrant sur le lit statique est de 85°C.

La teneur en lipides extractibles et en lipides totaux est déterminée par une méthode de dosage à l'hexane avec extraction au SOXHLET.

Le ratio lipides extractibles / lipides totaux est un indicateur de l'efficacité de l'émulsifiant : moins il y a de lipides extractibles et donc plus le ratio est faible, meilleure est l'émulsion, ce qui laisse présager une bonne stabilité du produit fini remis en suspension. Cet aspect est fondamental pour le côté appétence du produit.

De plus, les poudres sont caractérisées par un test d'écoulement qui consiste à calculer le temps d'écoulement d'une poudre dans un entonnoir dont les dimensions sont celles du test pharmacopée (test 2.9.16 de la Pharmacopée Européenne 6.0).

De manière plus précise, le test consiste à :

- Fixer ledit entonnoir à 20 cm de hauteur,
- Obstruer l'embout de l'entonnoir,
- Remplir l'entonnoir avec 100 g de poudre,
- Désobstruer l'embout et déclencher le chronomètre,
- Arrêter le chronomètre lorsque toute la poudre s'est écoulée,
- Relever le temps d'écoulement sur le chronomètre.

Les résultats obtenus sont présentés dans le Tableau VIII suivant :

Tableau VIII

| Nature de l'émulsifiant | % MS de la poudre | % lipides totaux | % lipides extractibles | Ratio LE / LT (%) | Test d'écoulement (s) |
|---|---|---|---|---|---|
| Protéine de pois native | 99,0 | 42,9 | 26,2 | 61 | Pas d'écoulement |
| Hydrolysat de protéines de pois conforme à l'invention | 98,6 | 41,9 | 6,3 | 15 | 14 |

**Exemple 8 : Analyse sensorielle**

Un profil sensoriel a été réalisé par la société Demanderesse de la façon suivante : les échantillons sont préparés dans des fioles en verre coloré à raison de 5 g de produit dans 150 g d'eau et maintenus à 50°C, ils sont ensuite présentés en aveugle aux panélistes.
Il a été demandé aux 10 panélistes qui ont participé au test de sentir et gouter le produit pour ensuite cocher le ou les descripteurs correspondant à chaque échantillon selon les catégories de descripteurs proposées.
Les résultats sont repris ci-dessous dans le tableau IX

Tableau IX

| **Critère olfactif :** pourcentage de citation des descripteurs | | |
|---|---|---|
| Descripteurs | Protéine de pois native | Hydrolysat de protéines de pois conforme à l'invention |
| Terre - béton - poussière | 30% | 20% |
| Paille - céréales | 0% | 10% |
| Amidon - papier - farine - fécule - carton | 30% | 10% |
| Pomme de terre | 10% | 10% |
| Pois - gluten - protéines | 40% | 10% |
| Acide - amer - âcre - piquant - fermenté | 60% | 50% |

| **Critère gustatif :** pourcentage de citation des descripteurs | | |
|---|---|---|
| Descripteurs | Protéine de pois native | Hydrolysat de protéines de pois conforme à l'invention |
| Terre - béton - poussière | 10% | 10% |
| Paille - céréales | 10% | 20% |
| Amidon - papier - farine - fécule - carton | 50% | 70% |
| Pomme de terre | 0% | 0% |
| Pois - gluten - protéines | 70% | 20% |
| Acide - amer - âcre - piquant - fermenté | 40% | 20% |

Ce profil montre que la flaveur des hydrolysats alcalins de l'invention est différente des protéines de pois.
Selon le panel d'experts, que ce soit sur le critère olfactif ou le critère gustatif, les descripteurs « pois », mais aussi « acide », « amer », « âcre », « piquant », et « fermenté » des hydrolysats sont atténués par rapport à ceux des protéines de pois natives.

**Exemple 9 (comparatif)** : **Préparation d'hydrolysats de protéines végétales selon d'autres procédés**

**Exemple 9-1 :**

On a préparé des hydrolysats de protéines de pois, de pomme de terre et de maïs en suivant le mode opératoire exemplifié dans le document GB 705 489, comprenant les étapes successives de :

1) préparation d'une solution de protéine à 10-15% de matière sèche (10% pour la protéine de pois, 13% pour les protéines de pomme de terre et de maïs),
2) ajout de 25 ml de NaOH à 40%
3) chauffage pendant 30 minutes à 82°C
4) neutralisation à pH = 7 avec HCl
5) chauffage pendant 2 minutes à 82°C
6) lyophilisation.

Les résultats obtenus sont rassemblés dans le Tableau X ci-dessous :

Tableau X

|  | Selon l'invention | Essai comparatif | | |
|---|---|---|---|---|
|  |  | Pois | Pomme de terre | Maïs |
| Richesse | 60-95 | 77,6 | 51 | 64,4 |
| Solubilité / sec | 60-100 | 99,5 | 83,3 | 65,5 |
| Degré d'hydrolyse | 5-8,5* | 4 | 7 | 2 |
| Capacité émulsifiante | 60-90 | 86,7 | 76 | 66,7 |
| Longueur de chaîne | 10-20 | 21 | 20 | 54 |
| * voir le Tableau V ci-dessus | | | | |

Le procédé selon GB 705 489 ne permet donc pas d'obtenir des hydrolysats de protéines végétales possédant le bon compromis de propriétés recherché selon l'invention.

**Exemple 9-2** :

On a préparé des hydrolysats de protéines de pois, de pomme de terre et de maïs en suivant le mode opératoire exemplifié dans le document US 2 999 753, comprenant les étapes successives de :

1) préparation d'une solution de protéine à 10-15% de matière sèche (10% pour la protéine de pois, 12% pour les protéines de pomme de terre et de maïs),
2) ajustement du pH à 10,7 avec NaOH
3) chauffage pendant 20 h à 40°C
4) neutralisation à pH = 5,5 avec HCl
5) lyophilisation.

Les résultats obtenus sont rassemblés dans le Tableau XI ci-dessous :

Tableau XI

|  | Selon l'invention | Essai comparatif | | |
|---|---|---|---|---|
|  |  | Pois | Pomme de terre | Maïs |
| Richesse | 60-95 | 79,9 | 54,4 | 66,9 |
| Solubilité / sec | 60-100 | 28,95 | 35,1 | 17,7 |
| Degré d'hydrolyse | 5-8,5* | 1 | 4 | 1 |
| Capacité émulsifiante | 60-90 | 57,3 | 60 | 58,3 |

(suite)

|  | Selon l'invention | Essai comparatif | | |
| --- | --- | --- | --- | --- |
|  |  | Pois | Pomme de terre | Maïs |
| Longueur de chaîne | 10-20 | 102 | 87 | 160 |
| * voir le Tableau V ci-dessus | | | | |

Le procédé selon US 2 999 753 ne permet donc pas d'obtenir des hydrolysats de protéines végétales possédant le bon compromis de propriétés recherché selon l'invention.

**Revendications**

1. Procédé de préparation d'hydrolysats alcalins de protéines végétales, **caractérisé en ce qu'**il comprend les étapes suivantes :

    1) préparer une suspension de protéines végétales choisies dans le groupe constitué des protéines du pois, des protéines de la pomme de terre et des protéines du maïs, à une matière sèche comprise entre 10 et 15%,
    2) ajuster le pH, sous agitation, à une valeur comprise entre 9,5 et 10,5 en utilisant, comme seul agent alcalin, un ou plusieurs hydroxydes alcalins choisis dans le groupe constitué de l'hydroxyde de sodium et de l'hydroxyde de potassium,
    3) chauffer la suspension ainsi obtenue à une température comprise entre 70 et 80°C, pendant 4 à 6 heures,
    4) neutraliser ladite suspension chauffée à l'aide d'un acide minéral, de préférence de l'acide chlorhydrique,
    5) sécher la suspension neutralisée de manière à obtenir l'hydrolysat alcalin.

2. Hydrolysats alcalins de protéines végétales, susceptibles d'être obtenus par le procédé de la revendication 1, **caractérisés en ce qu'**ils présentent :

    - une valeur de solubilité dans l'eau à pH 7,5 comprise entre 60 et 100%, de préférence entre 80 et 98%,
    - une capacité émulsifiante comprise entre 60 et 90%, de préférence entre 65 et 85%,
    - une longueur moyenne de chaîne peptidique comprise entre 10 et 20 aminoacides,
    - une richesse comprise entre 60 et 95%, préférentiellement comprise entre 80 et 85%.

3. Hydrolysats selon l'une quelconque des revendications 1 et 2, **caractérisés en ce qu'**ils présentent une capacité moussante comprise entre 150 et 250%.

4. Utilisation des hydrolysats alcalins selon l'une quelconque des revendications 2 et 3, ou susceptibles d'être obtenus par la mise en oeuvre du procédé de la revendication 1, comme agents émulsifiants dans les domaines des industries alimentaires humaines ou animales, pharmaceutiques, cosmétiques et industries chimiques, en particulier dans le domaine alimentaire.

5. Composition alimentaire contenant des hydrolysats alcalins selon l'une quelconque des revendications 2 et 3, ou susceptibles d'être obtenus par la mise en oeuvre du procédé de la revendication 1.

6. Composition alimentaire selon la revendication 5, **caractérisée par le fait qu'**il s'agit d'une émulsion émulsifiée par lesdits hydrolysats alcalins.

**Patentansprüche**

1. Verfahren zur Herstellung von alkalischen Hydrolysaten von Pflanzenproteinen, **dadurch gekennzeichnet, dass** es die folgenden Schritte umfasst:

    1) Herstellen einer Suspension von Pflanzenproteinen ausgewählt aus der Gruppe bestehend aus Erbsen-, Kartoffel- und Maisproteinen, mit einer Trockenmasse zwischen 10 und 15%,
    2) Einstellen des pH-Wertes unter Rühren auf einen Wert zwischen 9,5 und 10,5 unter Verwendung eines oder mehrerer Alkalimetallhydroxide als einziges alkalisches Mittel, ausgewählt aus der Gruppe bestehend aus Natrium- und Kaliumhydroxid,

3) Erhitzen der resultierenden Suspension bei einer Temperatur zwischen 70 und 80 °C für 4 bis 6 Stunden,

4) Neutralisieren der erhitzten Suspension mithilfe einer Mineralsäure, vorzugsweise Salzsäure,

5) Trocknen der neutralisierten Suspension, um das alkalische Hydrolysat zu erhalten.

2. Alkalische Hydrolysate von Pflanzenproteinen, welche durch das Verfahren nach Anspruch 1 erhältlich sind, **dadurch gekennzeichnet, dass** sie aufweisen:

- einen Löslichkeitswert in Wasser bei pH 7,5 zwischen 60 und 100 %, vorzugsweise zwischen 80 und 98%,
- eine Emulgierungsfähigkeit zwischen 60 bis 90%, vorzugsweise zwischen 65 und 85%,
- eine durchschnittliche Peptidkettenlänge zwischen 10 und 20 Aminosäuren,
- eine Güte zwischen 60 und 95%, vorzugsweise zwischen 80 und 85%.

3. Hydrolysate nach einem der Ansprüche 1 und 2, **dadurch gekennzeichnet, dass** sie eine Schäumungskapazität zwischen 150 und 250% aufweisen.

4. Verwendung von alkalischen Hydrolysaten nach einem der Ansprüche 2 und 3, oder erhältlich durch die Durchführung des Verfahrens nach Anspruch 1, als Emulgatoren auf dem Gebiet der menschlichen oder tierischen Lebensmittel-, Pharmazie-, Kosmetik- und Chemie-Industrie, insbesondere im Lebensmittelbereich.

5. Nahrungsmittelzusammensetzung, welche alkalische Hydrolysate nach einem der Ansprüche 2 und 3, oder erhältlich durch die Durchführung des Verfahrens nach Anspruch 1, umfasst.

6. Nahrungsmittelzusammensetzung nach Anspruch 5, **dadurch gekennzeichnet, dass** es sich um eine durch die alkalischen Hydrolysate emulgierte Emulsion handelt.

## Claims

1. A method for preparing alkaline hydrolysates of plant proteins, **characterized in that** it comprises the following steps:

1) preparing a suspension of plant proteins selected from the group consisting of pea proteins, potato proteins and corn proteins, with a dry matter content between 10 and 15%,

2) adjusting the pH, with stirring, to a value between 9.5 and 10.5 using, as the only alkaline agent, one or more alkali metal hydroxides selected from the group consisting of sodium hydroxide and potassium hydroxide,

3) heating the suspension thus obtained at a temperature between 70 and 80°C, for 4 to 6 hours,

4) neutralizing said heated suspension by means of a mineral acid, preferably hydrochloric acid,

5) drying the neutralized suspension to obtain the alkaline hydrolysate.

2. Alkaline hydrolysates of plant proteins, obtainable by the method of claim 1, **characterized in that** they have:

- a value of water solubility at pH 7.5 between 60 and 100%, preferably between 80 and 98%,
- an emulsifying capacity between 60 and 90%, preferably between 65 and 85%,
- an average length of peptide chain between 10 and 20 amino acids,
- a richness between 60 and 95%, preferably between 80 and 85%.

3. The hydrolysates as claimed in either one of claims 1 and 2, **characterized in that** they have a foaming capacity between 150 and 250%.

4. The use of the alkaline hydrolysates as claimed in either one of claims 2 and 3, or obtainable by implementing the method of claim 1, as emulsifiers in the sectors of human or animal food industries, the pharmaceutical industry, the cosmetics industry and chemical industries, in particular in the food sector.

5. A food composition containing alkaline hydrolysates as claimed in either one of claims 2 and 3, or obtainable by implementing the method of claim 1.

6. The food composition as claimed in claim 5, **characterized in that** it is an emulsion emulsified by said alkaline hydrolysates.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2522050 A
- GB 670413 A
- EP 1327390 A
- WO 9525437 A
- EP 1909592 A
- WO 2008001183 A
- WO 2007079458 A
- WO 2008110515 A
- GB 705489 A
- US 2999753 A